(19) Europäisches Patentamt
European Patent Office
Office européen des brevets

(11) Publication number: 0 384 432 B1

(12) # EUROPEAN PATENT SPECIFICATION

(45) Date of publication of patent specification: **08.12.93** (51) Int. Cl.⁵: **C09K 19/34**

(21) Application number: **90103335.7**

(22) Date of filing: **21.02.90**

(54) **Liquid crystalline compound and use thereof.**

(30) Priority: **22.02.89 JP 42535/89**
**19.09.89 JP 244336/89**

(43) Date of publication of application:
**29.08.90 Bulletin 90/35**

(45) Publication of the grant of the patent:
**08.12.93 Bulletin 93/49**

(84) Designated Contracting States:
**CH DE FR GB LI**

(56) References cited:
**CH-A- 655 324**
**DE-A- 3 017 499**
**US-A- 4 676 925**

(73) Proprietor: **DAISO CO., LTD.**
**10-8, Edobori 1-chome**
**Nishi-ku**
**Osaka-shi Osaka-fu(JP)**

(72) Inventor: **Kitamura, Tohru**
**441-1, Shinmachi 7-chome,**
**Fushimi-ku**
**Kyoto-shi, Kyoto-fu(JP)**
Inventor: **Sakaguchi, Kazuhiko**
**7-1-211, Minamisakurazuka 2-chome**
**Toyonaka-shi, Osaka-fu(JP)**
Inventor: **Shiomi, Yutaka**
**81, Motohama-cho 2-chome**
**Amagasaki-shi, Hyogo-ken(JP)**
Inventor: **Takehira, Yoshikazu**
**5-4, Suzuhara-cho 5-chome**
**Itami-shi, Hyogo-ken(JP)**

(74) Representative: **VOSSIUS & PARTNER**
**Postfach 86 07 67**
**D-81634 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

This invention relates to liquid crystalline compound and a liquid crystal composition comprising said compound, which is useful as an element for display devices or an element for opto-electronics devices.

Liquid crystals have widely been used as a material for display devices, where TN (Twisted Nematic) type display system is usually employed. This TN display system has such advantages that it has less electric consumption and that it gives less eye fatigue because it is a receptor type, but on the other hand, this system is disadvantageous in that the driving force is very weak because it is driven mainly on the basis of anisotropy of relative dielectric constant and it is slow in response speed, and hence, this system can not be applied to the devices which require high response speed.

It is also known that opto-electronics devices comprise a layer (cell) containing a liquid crystal composition as the main component and two electrodes which are provided on both sides of the layer of a liquid crystal composition (cf. N.A. Clark et al., Appl. Phys. Lett. 36(11), pp. 899-901 (1 June 1980); and N.A. Clark et al., Mol. Cryst. Liq. Cryst., 1983, Vol. 94, pp. 213-233).

Liquid crystals having ferroelectricity have first been found by R.B. Meyer et al. in 1975 (cf. J. Physique, 36, L-69, 1975). This liquid crystal is driven by a comparatively large force derived from spontaneous polarization and shows extremely high response speed and has also good memory. Owing to such excellent properties, the ferroelectric liquid crystals have been noticed as a new type of display element. In order to exhibit the ferroelectricity, the liquid crystalline compounds should show chiral smectic C phase (SmC* phase) and thus should contain at least one asymmetric carbon atom in the molecule. It is also necessary to have a dipole moment in the direction vertical to the long axis of the molecule.

A ferroelectric liquid crystal DOBAMBC synthesized by Meyer et al. has the following formula:

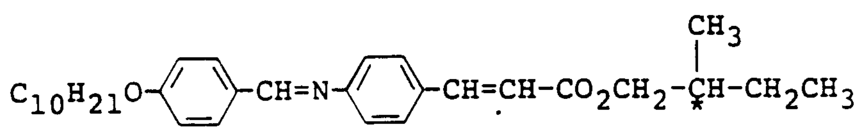

and satisfies the above conditions, but it contains a Schiff base and hence is chemically unstable and show such a low spontaneous polarization as 3 x $10^{-9}$ $C/cm^2$. Since then, there have been synthesized many ferroelectric liquid crystalline compounds, but any practically useful compound having sufficiently high response speed has never been found.

Among the known ferroelectric liquid crystalline compounds, DOBA-1-MBC which has the asymmetric carbon atom at the position nearer to the carbonyl group than in DOBAMBC and has the following formula:

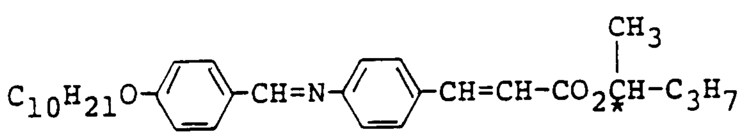

shows a spontaneous polarization of 5 x $10^{-8}$ $C/cm^2$ which is larger than that of DOBAMBC. It is assumed that this will be caused by the following difference. That is, the asymmetric carbon atoms and the dipole which are important factors for the appearance of ferroelectricity are positioned close to each other, and thereby, the free rotation of the dipole moiety of molecule is restricted and then the orientation of the dipole is increased. Thus, it is assumed that the known ferroelectric liquid crystalline compounds can not give satisfactory spontaneous polarization and high response speed because the asymmetric carbon atom having an inhibitory action on the free rotation of molecule is present on the linear chain in the known ferroelectric liquid crystalline compounds and hence the free rotation of molecule can not completely be inhibited and the dipole moiety can not be fixed.

It is the object of the invention to provide novel liquid crystalline compounds which exhibit ferroelectricity and are chemically stable. This object could be achieved on the basis of the finding that the free rotation of the crystalline compounds can be inhibited by providing a compound wherein the asymmetric carbon atom is contained in a 5-membered lactone ring, by which there can be obtained a chemically stable liquid crystalline compound having ferroelectricity.

Another object of the invention is to provide a liquid crystal composition which comprises at least one of said liquid crystalline compounds, i.e. compounds having optically active γ-lactone ring in the molecule

wherein two asymmetric carbon atoms are present in the 5-membered lactone ring, or compounds having a specific substituent on the phenyl ring of the above compounds.
Still another object of the invention is to provide an element for opto-electronics devices comprising said liquid crystal composition.

The liquid crystalline compound of this invention contains an optically active γ-lactone ring and has the following formula (I):

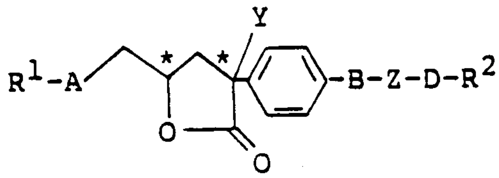

(I)

wherein $R^1$ and $R^2$ are each independently an alkyl group having 1 to 15 carbon atoms or an alkenyl group having 2 to 15 carbon atoms; A and D are each independently a single bond or -O-;
B is a single bond or

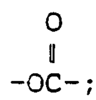

Z is a single bond or one of the groups

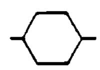 or 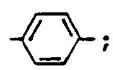

Y is hydrogen atom or $-CH_3$; and the symbol * is an asymmetric carbon atom with the proviso that the compound in which A is O, $R^1$ is $-CH_3$, Y is a hydrogen atom, B and Z are each a single bond, D is O and $R^2$ is $-CH_3$ is excluded. This invention also includes a liquid crystal composition comprising at least one of the above liquid crystalline compounds and an element for opto-electronics devices using said composition.

In the specification, the term "alkyl group" for $R^1$ and $R^2$ includes a straight chain or branched chain alkyl group having 1 to 15 carbon atoms, such as methyl, ethyl, n-propyl, n-butyl, n-pentyl, n-hexyl, n-heptyl, n-octyl, n-nonyl, n-decyl, n-undecyl, n-dodecyl, n-tridecyl, n-tetradecyl, n-pentadecyl, isopropyl, t-butyl, 2-methylpropyl, 1-methylpropyl, 3-methylbutyl, 2-methylbutyl, 1-methylbutyl, 4-methylpentyl, 3-methylpentyl, 2-methylpentyl, 1-methylpentyl, 5-methylhexyl, 4-methylhexyl, 3-methylhexyl, 2-methylhexyl, 1-methylhexyl, 6-methylheptyl, 5-methylheptyl, 4-methylheptyl, 3-methylheptyl, 2-methylheptyl, 1-methylheptyl, 7-methyloctyl, 6-methyloctyl, 5-methyloctyl, 4-methyloctyl, 3-methyloctyl, 2-methyloctyl, 1-methyloctyl, 8-methylnonyl, 7-methylnonyl, 6-methylnonyl, 5-methylnonyl, 4-methylnonyl, 3-methylnonyl, 2-methylnonyl, 1-methylnonyl, 3,7-dimethyloctyl and 3,7,11-trimethyldodecyl.

The term "alkenyl group" for $R^1$ and $R^2$ includes a straight chain alkenyl group having 2 to 15 carbon atoms, such as vinyl, propenyl, butenyl, pentenyl, hexenyl, heptenyl, octenyl, nonenyl, decenyl, undecenyl, dodecenyl, tridecenyl, tetradecenyl, pentadecenyl, and a branched chain alkenyl group containing 4 to 15 carbon atoms, such as 1-methylpropenyl, 2-methylpropenyl, 3-methylpropenyl and 4-methylpropenyl.

The compounds of this invention contain a carbonyl group within the 5-membered ring and two asymmetric carbon atoms on the ring as a moiety having a dipole moment as an origin of ferroelectricity, and hence, the free rotation at this moiety is inhibited and thereby the dipole moiety is directed to one direction, which is effective for enlarging the spontaneous polarization and for increasing the response speed. The liquid crystalline compounds of this invention contain two asymmetric carbon atoms in the γ-lactone ring and hence there are present two kinds of diastereomer. These are all suitable for inhibition of free rotation of the dipole moiety, and they are used as a liquid crystal alone or in a mixture of two or more thereof. The liquid crystalline compounds of the invention include not only the compounds which are in the liquid crytalline state alone but also the compounds which do not take the liquid crystalline form by itself but still are useful as a component of a liquid crystal composition.

The compounds (I) of the invention can be prepared in the following manner.

When B in the formula (I) is a single bond, the compounds (I) of the invention can be prepared by the processes as shown in the following Reaction Scheme-I wherein $R^1$, $R^2$, A, D, Z, Y and the symbol * are the same as defined in the general formula (I), and LDA means lithium diisopropylamide and MeI means methyl iodide.

Reaction Scheme-I

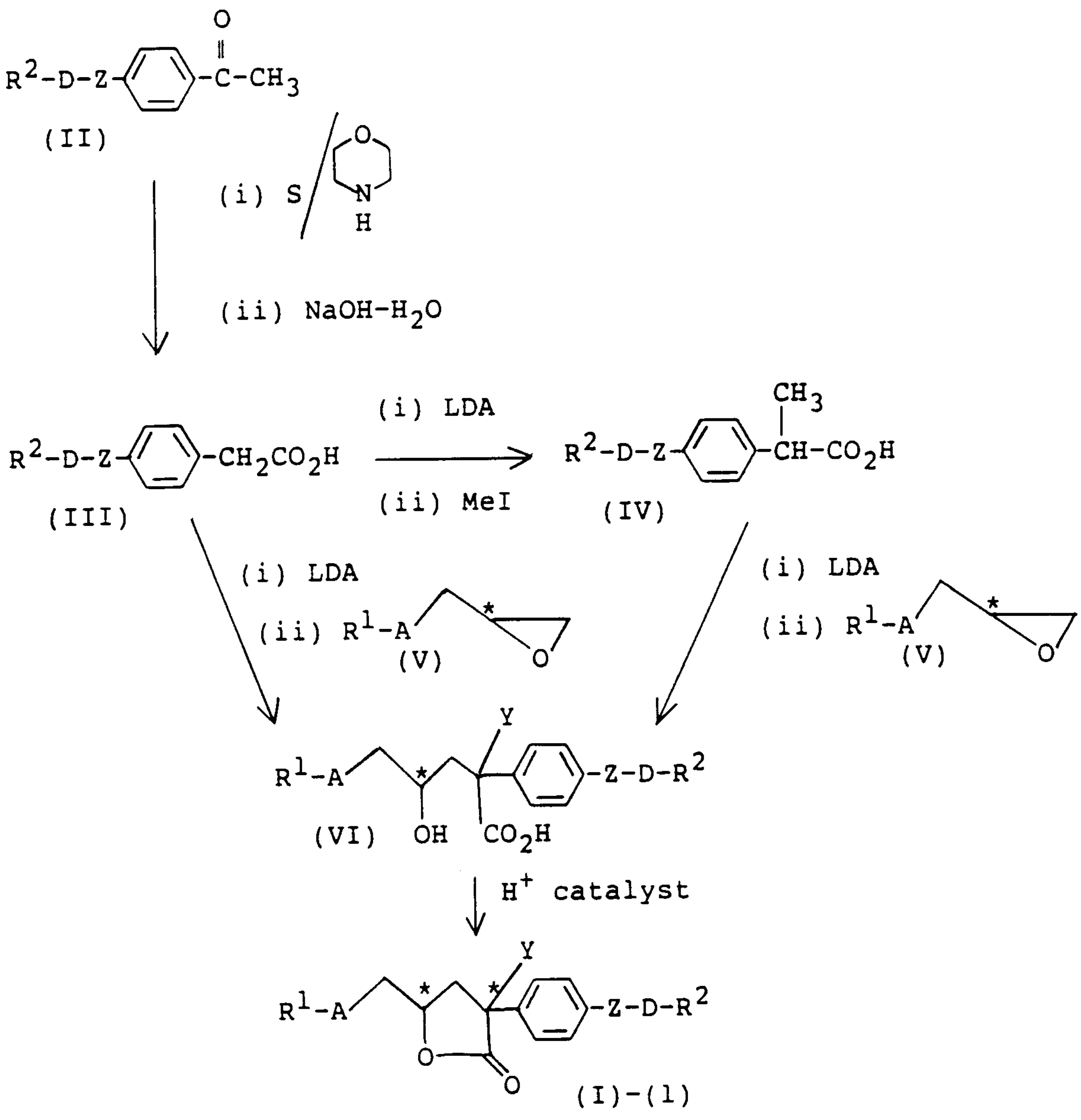


The acetophenone derivative (II) prepared by a known method is subjected to the Willgerodt reaction to produce the phenylacetic acid derivative (III). The derivative (III) is then reacted with two times molar amount of lithium diisopropylamide (LDA) at -30 to 10 °C and then with 0.3 to 3 times molar amount of the optically active epoxy compound (V) at -78 °C to room temperature to give the addition compound (VI). The compound (VI) is then dehydrated in the presence of an acid catalyst such as sulfuric acid, hydrochloric acid, paratoluenesulfonic acid in a solvent such as benzene or toluene to give the optically active γ-lactone derivative (I)-(1) which is the compound (I) of the invention-wherein B is a single bond.

Alternatively, the addition compound (VI) wherein Y is $-CH_3$ can be prepared by reacting the phenylacetic acid derivative (III) with lithium diisopropylamide (LDA) and then with methyl iodide (MeI) to give the phenylmethylacetic acid derivative (IV), followed by reacting with lithium diisopropylamide (LDA) and then with the optically active epoxy compound (V).

The optically active epoxy compound (V) used in the above reaction wherein A is a single bond, which is referred to (V)-(1), can be prepared by the following reaction wherein $R^1$ and the symbol * are the same as defined in the general formula (I) and X is a halogen atom.

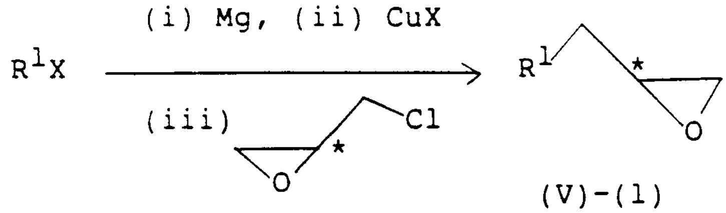


(V)-(1)

That is, an alkyl halide or alkenyl halide ($R^1X$) is reacted with magnesium to produce a Grignard reagent, which is then reacted with optically active epichlorohydrin in the presence of a copper halide (CuX) to give the optically active epoxy compound (V)-(1).

The optically active epoxy compound (V) wherein A is -O-, which is referred to (V)-(2), can be prepared by the following reaction wherein $R^1$ and the symbol * are the same as defined in the general formula (I), and R is methyl, ethyl, n-propyl, n-butyl and phenyl.

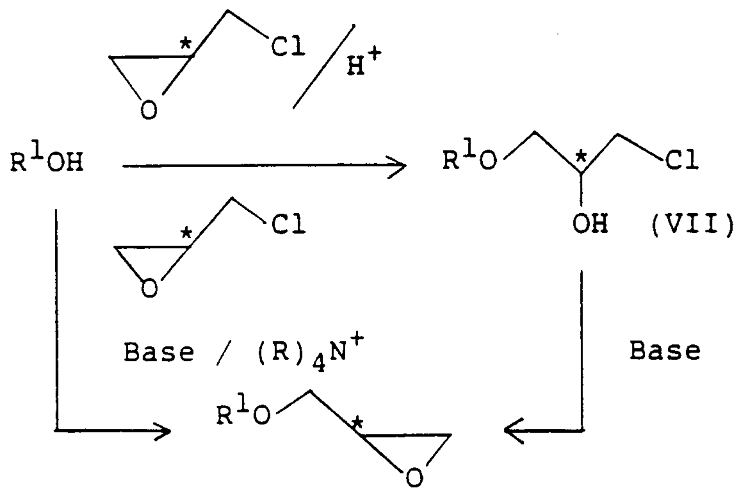


(VII)

(V)-(2)

That is, the compound (V)-(2) can be prepared by either a two step procedure which comprises reacting an alcohol ($R^1OH$) with optically active epichlorohydrin in the presence of an acid catalyst to give the chlorohydrin ether (VII) and then treating the ether (VII) with an alkali to form a closed ring, or a single step procedure which comprises reacting the alcohol ($R^1OH$) with optically active epichlorohydrin and a base in the presence of a catalyst of a quarternary ammonium salt.

Alternatively, the above optically active epoxy compound (V)-(1) can be prepared by the reaction of an olefin and air using microorganisms.

The optically active epichlorohydrin can be prepared in a high purity by the processes as described in JP-A-132196/1986 and 6697/1987 (as to R isomer) and by the process as described in JP-A-230567/1989 (as to S isomer).

The compound of the general formula (I) wherein B is

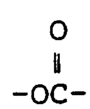

can be prepared by the following Reaction Scheme-II wherein $R^1$, $R^2$, A, D, Z, Y and the symbol * are the same as defined in the general formula (I) and Me, Et and ph represent methyl, ethyl and phenyl, respectively, and LDA and MeI are the same as mentioned above.

Reaction Scheme-II

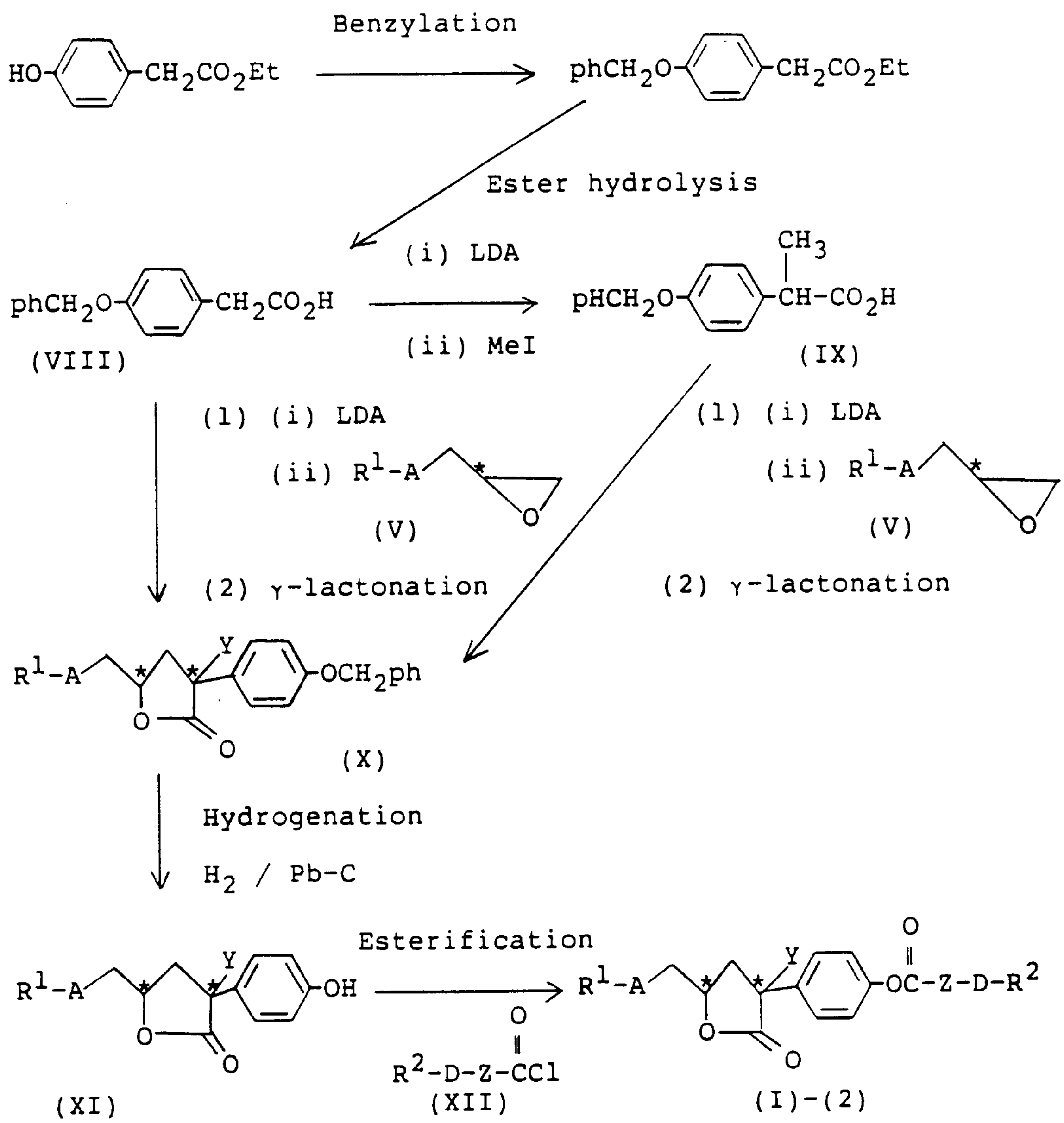


In the Reaction Scheme-II, p-hydroxyphenylacetic acid ethyl ester is reacted with a benzyl halide to produce the corresponding benzyl ether, which ester part is then hydrolyzed to give p-benzyloxyphenylacetic acid (VIII). The conversion of this compound (VIII) into γ-lactone derivative (X) or the conversion of this compound (VIII) into γ-lactone derivative (X) through the compound (IX) can be conducted in the same manner as in the process for producing the γ-lactone derivative (I)-(1) from the phenylacetic acid derivative (III) through the addition compound (VI) or the process for producing the γ-lactone derivative (I)-(1) from the phenylacetic acid derivative (III) through the phenylmethylacetic acid derivative (IV) and then the addition compound (VI) in the above Reaction Scheme-I. The obtained γ-lactone derivative (X) is hydrogenated in the presence of a catalyst to give 2-(p-hydroxyphenyl)-γ-lactone derivative (XI) and the compound (XI) is then esterified with the carboxylic acid chloride derivative (XII) to give the optically active γ-lactone derivative (I)-(2), which is the compound (I) wherein B is

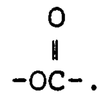

When the epoxy compound (V) used in the Reaction Schemes-I and -II is racemic, the final product of the $\gamma$-lactone derivatives (I)-(1) and (I)-(2) are racemic. This racemic compound can be added to other optically active liquid crystalline compounds in order to regulate the helical pitch thereof.

The liquid crystal composition of this invention may be obtained by mixing at least one of the compound (I) as prepared above with a chiral or non-chiral liquid crystal or a mixture thereof.

The chiral or non-chiral liquid crystals employed in the liquid crystal composition of this invention are not particularly limited but may be any conventional chiral or non-chiral liquid crystals which shows chiral smectic C phase after mixing with the compound (I).

The conventional liquid crystals include those described in e.g. Flüssige Kristalle in Tabellen I, VEB Deutscher Verlag für Grundstoffindustrie, Leipzig, 1974; Flüssige Kristalle in Tabellen II, VEB Deutscher Verlag für Grundstoffindustrie, Leipzig, 1984.

Typical example of the above chiral or non-chiral liquid crystals include the compound of the general formula (J):

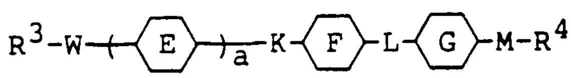 (J)

wherein E, F and G are each independently one of the 6-membered rings

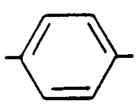, 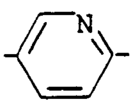, 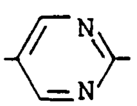, 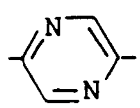, 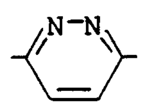, 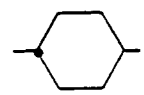,

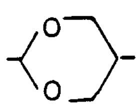 and 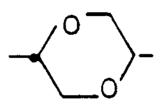,

the hydrogen atom(s) in the 6-membered ring being optionally substituted with a halogen atom, cyano group or nitro group; a is 0 or 1; W, K, L and M are each a single bond or one of the groups

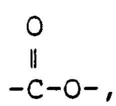,

$-CH_2O-$, $-C{\equiv}C-$, $-CH=CH-$, $-O-$, $-S-$,

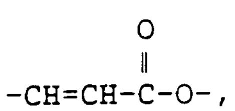, 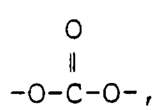,

$-N=CH-$ and $-CH_2-CH_2-$; provided that K is a single bond when $a=0$; $R^3$ and $R^4$ are each independently a straight chain or branched chain alkyl group having 1 to 15 carbon atoms, which may contain one or more asymmetric carbon atoms.

Particularly suitable examples of the chiral or non-chiral liquid crystal are a compound of the formula (J-1):

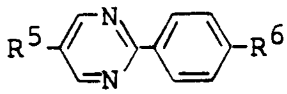 (J-1)

wherein $R^5$ and $R^6$ are the same or different and are each a straight chain or branched chain alkyl group having 1 to 15 carbon atoms or a straight chain or branched chain alkoxy group having 1 to 15 carbon atoms, said alkyl and alkoxy groups having optionally one or more asymmetric carbon atoms, and a compound of the formula (J-2):

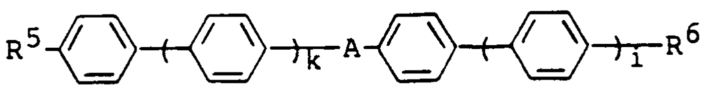 (J-2)

wherein $R^5$ and $R^6$ are as defined above, A is

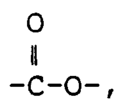

and k and i are independently 0 or 1, but $k + i \neq 2$.

The liquid crystal composition of this invention is useful for preparing a liquid crystal cell of an electrically controlled birefrigence mode or guest-host mode, which is prepared by attaching a transparent electrode to the liquid crystal composition of this invention, sandwiching the resultant electrode-attached liquid crystal composition with two sheets of glass plate which is surface-treated for orientation with a polymer (e.g. polyethylene, polyester, nylon, polyvinyl alcohol or polyimide), and providing a polarizer. The thus prepared liquid crystal cell can be used as an element for display devices or an element for opto-electronics devices. The liquid crystalline compound (I) of this invention have excellent heat stability and light stability, and the liquid crystal composition comprising the liquid crystalline compound (I) shows excellent properties as ferroelectric liquid crystal. The liquid crystal composition comprising the liquid crystalline compound (I) of this invention and nematic liquid crystal is also useful for the following utilities.

(1) Liquid crystal composition of TN (Twisted Nematic) type or STN (Super Twisted Nematic) type liquid crystals wherein the compound (I) is effective to inhibit occurrence of reverse domain.

(2) Display element utilizing cholesteric -nematic phase transfer effects (cf. J.J. Wysoki, A. Adams and W. Haas; Phys. Rev. Lett., 20, 1024, 1968).

(3) Display element utilizing White-Taylor type guest-host effects (cf. D.L. White and G.N. Taylor; J. Appl. Phys., 45, 4718, 1974).

(4) Notch filter or band-pass filter utilizing selective scattering effects by fixing the cholesteric phase in matrix (cf. F.J. Kahn; Appl. Phys. Lett., 18, 231, 1971).

(5) Circularly polarized light beam splitter utilizing circularly polarized light characteristics of the cholesteric phase (cf. S.D. Jacob; SPIE. 37, 98, 1981).

As mentioned above, the liquid crystalline compounds of this invention have a more excellent heat stability and light stability and higher chemical stability than those of the conventional liquid crystalline material and show excellent properties as ferroelectric liquid crystal, and hence, provide for liquid crystalline material with a more rapid response speed than that of the conventional liquid crystal composition.

This invention is illustrated by the following Preparations and Examples, but should not be construed to be limited thereto.

In Examples, the positions of R and S in the optically active compounds (I) of this invention are shown by the position numbers in the following formula:

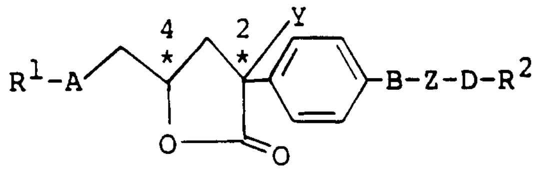

(I)

The phase transfer temperature in Examples was measured by DSC (Differential Scanning Colorimetry) and a polarizing microscope. Besides, the symbols in the phase transfer temperature mean as follows:

| | |
|---|---|
| C: | Crystalline phase |
| SmA: | Smectic A phase |
| SmC: | Smectic C phase |
| SmC*: | Chiral smectic C phase |
| Sm1: | Non-identified smectic phase other than SmA, SmC and SmC*. |
| N: | Nematic phase |
| N*: | Chiral nematic phase |
| I: | Isotropic liquid |
| Xn: | Non-identified phase |

<Preparation of the compound (V)>

Preparation 1

Preparation of (R)-1,2-epoxynonane:

A reaction vessel is charged with a suspension of cuprous iodide (1.9 g, 10 mmol) in ether (75 ml) and thereto is added Grignard reagent, prepared from hexyl bromide (12.38 g, 75 mmol) and magnesium (2 g, 82.5 mmol) in tetrahydrofuran (75 ml), under nitrogen stream at -30 °C and the mixture is stirred for 30 minutes. A solution of R-epichlorohydrin [4.63 g, 50 mmol, chemical purity: more than 98.5 %, optical purity: more than 99 % ($[\alpha]_D^{25}$ = -34.0 °, c = 1.2, methanol)] in tetrahydrofuran/ether (1/1)(100 ml) is added at the same temperature and the mixture is stirred for 2 hours. After completion of the reaction, a saturated aqueous ammonium chloride solution (100 ml) is added and the temperature is raised to room temperature. After extraction with ether, the extract is washed with a saturated saline and the organic layer is dried over anhydrous magnesium sulfate. Then, the solvent is ditilled off under reduced pressure and the residue is purified by distillation under reduced pressure to give (R)-chlorohydrin compound (6.29 g, 35.2 mmol, yield: 70 %) of the following formula:

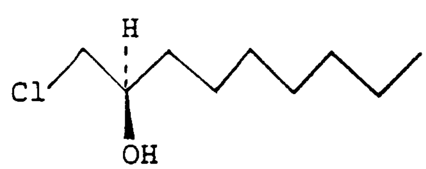

$[\alpha]_D^{25}$ = +8.20 ° (neat)
b.p. 60 to 66 °C (14 mmHg, Kugelrohr)
IR $\nu$max (neat): 3380 $cm^{-1}$
NMR ($CDCl_3$) $\delta$: 0.7-1.8 (15H, m), 2.2 (1H, brd), 3.3-3.9 (3H, m)

A mixture of the above (R)-chlorohydrin compound (4.99 g) and 48 % sodium hydroxide (50 ml) is refluxed with stirring for 2 hours. After completion of the reaction, the product is extracted with ether and the extract is purified by distillation to give (R)-1,2-epoxy-n-nonane (3.97 g).
$[\alpha]_D^{25}$ = +10.87 °
NMR ($CDCl_3$) $\delta$: 0.88 (3H, t), 1.2-1.6 (12H, m), 2.46 (1H, m), 2.74 (1H, m), 2.91 (1H, m)

Preparation 2

Preparation of R-methylglycidyl ether:

To a solution of sulfuric acid (0.98 g) in methanol (100 ml) refluxed with stirring is added dropwise a solution of the same (R)-epichlorohydrin (23.66 g) as used in Preparation 1 in methanol (20 ml) over 20 minutes and the mixture is refluxed with stirring for further 20 minutes. After cooling the reaction mixture to 10-15 °C, a solution of potassium hydroxide (31 g) in methanol (150 ml) is added dropwise and the mixture is stirred for 10 minutes. The reaction solution is transferred into a saturated saline and the product is extracted with methylene chloride and the extract is purified by ditillation (b.p. 110 °C) to give (R)-methylglycidyl ether (4.14 g).
$[\alpha]_D^{28}$ = +6.49 ° (c = 1.086, $CH_2Cl_2$)
NMR ($CDCl_3$) δ: 2.63 (1H, dd), 2.82 (1H, m), 3.16 (1H, m), 3.34 (1H, dd), 3.42 (3H, s), 3.71 (1H, dd)

Preparation 3

Preparation of (R)- and (S)-n-hexylglycidyl ethers:

To a mixture of 50 % sodium hydroxide (40 g), the same (R)-epichlorohydrin (24 g) as used in Preparation 1 and tetrabutyl ammonium hydrogen sulfate (400 mg) is added dropwise n-hexanol (6 ml) while cooling at 20-25 °C. After stirring the reaction mixture at the same temperature for additional 3 hours, water is added to the mixture and the product is extracted with ether. The extract is purified by distillation under reduced pressure to give (R)-n-hexylglycidyl ether (3.35 g).
$[\alpha]_D^{37}$ = +2.48 ° (c = 1.048, $CH_2Cl_2$)
b.p. 52 °C / 4 mmHg
NMR ($CDCl_3$) δ: 0.89 (3H, m), 1.2-1.4 (6H, m), 1.58 (2H, m), 2.58 (1H, dd), 2.77 (1H, dd), 3.12 (1H, m), 3.36 (1H, dd), 3.48 (2H, m), 3.70 (1H, dd)

The same procedures as mentioned above are repeated except that (S)-epichlorohydrin [chemical purity: more than 98.5 %, optical purity: more than 99 % ($[\alpha]_D^{25}$ = +34.0 °, c = 1.2, methanol)] is employed instead of (R)-epichlorohydrin to give (S)-n-hexylglycidyl ether(3.20 g).
$[\alpha]_D^{37}$ = -2.45 ° (c = 1.005, $CH_2Cl_2$)
NMR ($CDCl_3$) δ: 0.89 (3H, m), 1.2-1.4 (6H, m), 1.58 (2H, m), 2.58 (1H, dd), 2.77 (1H, dd), 3.12 (1H, m), 3.36 (1H, dd), 3.48 (2H, m), 3.70 (1H, dd)

Preparation 4

Preparation of (S)-allylglycidyl ether:

To a solution of sulfuric acid (0.5 g) in allyl alcohol (100 ml) refluxed with stirring is added dropwise a solution of the same (S)-epichlorohydrin (19.54 g) as used in Preparation 3 in allyl alcohol (20 ml) over 20 minutes and the mixture is refluxed with stirring for additional 20 minutes. After cooling the reaction solution to 10-15 °C, a solution of potassium hydroxide (25.2 g) in methanol (130 ml) is added dropwise and the mixture is stirred for 10 minutes. The reaction solution is transferred into a saturated saline and the product is extracted with methylene chloride and the extract is purified by distillation to give (S)-allylglycidyl ether (9.51 g).
$[\alpha]_D^{30}$ = -9.24 ° (c = 1.075, $CH_2Cl_2$)
NMR ($CDCl_3$) δ:2.61 (1H, dd), 2.80 (1H, t), 3.16 (1H, m), 3.40 (1H, dd), 3.73 (1H, dd), 4.05 (1H, m), 5.20 (1H, d), 5.29 (1H, d), 5.91 (1H, m)

<Preparation of the compound (III)>

Preparation 5

Preparation of 4-(4′-n-heptyl)-biphenylacetic acid:

A mixture of 4-acetyl-4′-n-heptylbiphenyl (10.85 g) and sulfur (2.36 g) in morpholine (20 ml) is refluxed with stirring for 9 hours. To the reaction solution is added a solution of sodium hydroxide (29.5 g) in water (80 ml) and ethanol (100 ml) and the mixture is stirred for 9 hours. The reaction solution is then transferred

into water and the mixture is acidified with hydrochloric acid. The precipitated solid is filtered off to give a crude product (13.51 g). The obtained crude product is purified by silica gel column chromatography to give the desired compound (8.29 g).
m.p. 154 to 162 ° C
IR (Nujol): 1724 $cm^{-1}$
NMR ($CDCl_3$) δ: 0.88 (3H, m), 1.2-1.4 (8H, m), 1.64 (2H, m), 2.63 (2H, t), 3.68 (2H, s), 7.23 (2H, d), 7.33 (2H, d), 7.48 (2H, d), 7.54 (2H, d)

Preparation 6

Preparation of 4-(4′-n-nonyloxy)-biphenylacetic acid:

A mixture of 4-acetyl-4′-n-nonyloxybiphenyl (10.14 g) and sulfur (1.536 g) in morpholine (20 ml) is refluxed with stirring for 15 hours. To the reaction solution is added a solution of sodium hydroxide (25 g) in water (65 ml) and ethanol (100 ml) and the mixture is stirred for 9 hours. The reaction solution is then transferred into water and the mixture is acidified with hydrochloric acid. The precipitated solid is filtered off to give a crude product. The obtained crude product is purified by silica gel column chromatography to give the desired compound (12.84 g).
m.p. 175 to 176 ° C
IR (Nujol): 1704 $cm^{-1}$
NMR ($CDCl_3$) δ:0.89 (3H, m), 1.2-1.5 (12H, m), 1.80 (2H, m), 3.69 (2H, s), 3.99 (2H, t), 6.95 (2H, d), 7.33 (2H, d), 7.49 (2H, d), 7.52 (2H, d)

Preparation 7

Preparation of 4-(4′-n-butyltranscyclohexyl)phenylacetic acid:

A mixture of 4-(4′-n-butyltranscyclohexyl)acetophenone (5 g) and sulfur (1.24 g) in morpholine (7.5 ml) is refluxed with stirring for 11 hours. To the reaction solution is added a solution of sodium hydroxide (16.7 g) in water (43.4 ml) and ethanol (67 ml) and the mixture is stirred for 7 hours. The reaction solution is then transferred into water, the mixture is acidified with hydrochloric acid and the product is extracted with ether. The extracted crude product is purified by silica gel column chromatography to give the desired compound (3.33 g).
m.p. 72 to 74 ° C
IR (Nujol): 1718 $cm^{-1}$
NMR ($CDCl_3$) δ: 0.8-1.5 (14H, m), 1.86 (4H, m), 2.44 (1H, t), 3.59 (2H, s), 7.17 (4H, m)

<Preparation of the compound (IV)>

Preparation 8

Preparation of 2-(4′-nonyloxy-4-biphenyl)propionic acid:

To a solution of diisopropylamine (506 mg) in tetrahydrofuran (10 ml) cooled at -78 ° C is added dropwise a solution of n-butyllithium in hexane (3 ml) and the temperature is raised gradually to 0 ° C and the mixture is stirred for 30 minutes. To this reaction solution is added dropwise a solution of 4-(4′-n-nonyloxy)-biphenylacetic acid (708 mg) prepared in Preparation 6 in tetrahydrofuran (8 ml) and the mixture is stirred for 1 hour. The reaction solution is cooled to -78 ° C and a solution of methyl iodide (426 mg) in tetrahydrofuran (2 ml) is added dropwise. After the temperature of the reaction solution is gradually raised to room temperature and the reaction solution is stirred for 6 hours, water is added and the mixture is acidified with hydrochloric acid, followed by extraction of the product with chloroform to give the desired compound (730 mg).
NMR ($CDCl_3$) δ: 0.89 (3H, t), 1.2-1.6 (12H, m), 1.55 (3H, d), 1.80 (2H, m), 3.79 (1H, q), 3.99 (2H, t), 6.95 (2H, d), 7.37 (2H, d), 7.4-7.6 (4H, m)

Example 1

To a solution of diisopropylamine (113 mg) in tetrahydrofuran (2 ml) cooled at -78°C is added dropwise a 15 % solution of n-butyllithium in hexane (0.7 ml) and the temperature is raised gradually to 0°C and the mixture is stirred for 30 minutes. To this reaction solution is added dropwise a solution of 4-(4'-n-nonyloxy)-biphenylacetic acid (177 mg) prepared in Preparation 6 in tetrahydrofuran (2 ml) and the mixture is stirred for 1 hour. The reaction solution is cooled to -78°C and a solution of (R)-1,2-epoxynonane (85 mg) prepared in Preparation 1 in tetrahydrofuran (2 ml) is added dropwise. After the temperature of the reaction solution is gradually raised to room temperature and the reaction solution is stirred for 6 hours, water is added and the mixture is acidified with hydrochloric acid, followed by extraction of the product with chloroform. To the extract is added dry benzene and a catalytic amount of sulfuric acid and the mixture is stirred with heating for 6 hours while draining benzene in portions. After cooling, the benzene is distilled off under reduced pressure and the residue is purified by silica gel column chromatography to give γ-lactone derivatives (2R, 4R)(35 mg) and (2S, 4R)(104 mg) of the following formulas, respectively.

(2R, 4R) isomer:

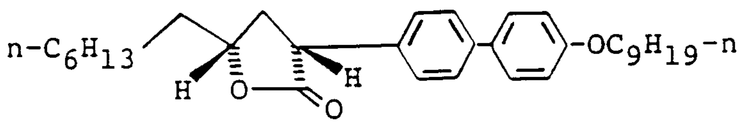

Phase transfer temperature:

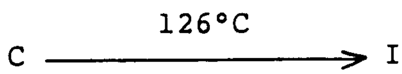


$[\alpha]_D^{34}$ = -5.42° (c = 1.66, $CH_2Cl_2$)

NMR ($CDCl_3$) δ: 0.89 (6H, m), 1.2-1.9 (26H, m), 2.05 (1H, td), 2.74 (1H, ddd), 3.88 (1H, dd), 3.98 (2H, t), 4.48 (1H, m), 6.95 (2H, d), 7.31 (2H, d), 7.48 (2H, d), 7.53 (2H, d) IR (Nujol): 1750 $cm^{-1}$

(2S, 4R) isomer:

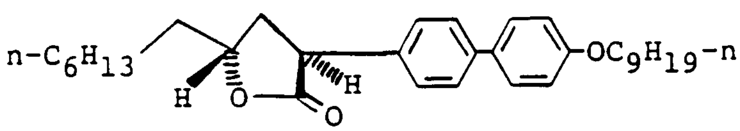

Phase transfer temperature:

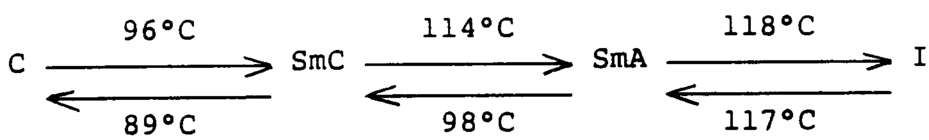


$[\alpha]_D^{34}$ = +29.33° (c = 0.95, $CH_2Cl_2$)

NMR ($CDCl_3$) δ: 0.89 (6H, m), 1.2-1.9 (26H, m), 2.41 (1H, ddd), 2.53 (1H, dt), 3.93 (1H, dd), 3.99 (2H, t), 4.66 (1H, m), 6.96 (2H, d), 7.32 (2H, d), 7.48 (2H, d), 7.53 (2H, d) IR (Nujol): 1750 $cm^{-1}$

Example 2

To a solution of diisopropylamine (339 mg) in tetrahydrofuran (6 ml) cooled at -78°C is added dropwise a 15 % solution of n-butyllithium in hexane (2.1 ml) and the temperature is raised gradually to 0°C and the mixture is stirred for 30 minutes. To this reaction solution is added dropwise a solution of 4-(4'-n-nonyloxy)-biphenylacetic acid (531 mg) prepared in Preparation 6 in tetrahydrofuran (5 ml) and the mixture is stirred

for 1 hour. The reaction solution is cooled to -78 °C and a solution of (R)-n-hexylglycidyl ether (256 mg) prepared in Preparation 3 in tetrahydrofuran (1 ml) is added dropwise. After the temperature of the reaction solution is gradually raised to room temperature and the reaction solution is stirred for 6 hours, water is added and the mixture is acidified with hydrochloric acid, followed by extraction of the product with chloroform. To the extract is added dry benzene and a catalytic amount of sulfuric acid and the mixture is stirred with heating for 6 hours while draining benzene in portions. After cooling, the benzene is distilled off under reduced pressure and the residue is purified by silica gel column chromatography to give γ-lactone derivatives (2R, 4S)(259 mg) and (2S, 4S) (207 mg) of the following formulas, respectively.

(2R, 4S) isomer:

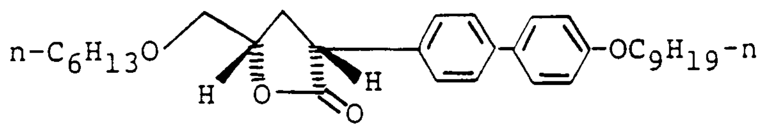

Phase transfer temperature:

$$C \xrightarrow{92°C} I$$

$[\alpha]_D^{23}$ = +1.36 ° (c = 1.06, $CH_2Cl_2$)

NMR ($CDCl_3$) δ: 0.89 (6H, m), 1.2-1.5 (18H, m), 1.58 (2H, m), 1.78 (2H, m), 2.32 (1H, td), 2.66 (1H, ddd), 3.51 (2H, t), 3.61 (1H, dd), 3.70 (1H, dd), 3.89 (1H, dd), 3.97 (2H, t), 4.63 (1H, m), 6.95 (2H, d), 7.23 (2H, d), 7.48 (2H, d), 7.52 (2H, d)

(2S, 4S) isomer:

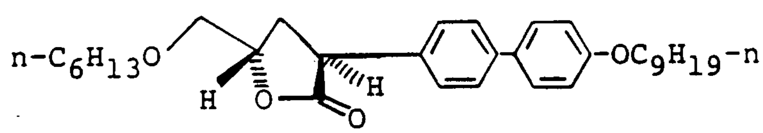

Phase transfer temperature:

$$C \xrightarrow{64°C} I$$

$[\alpha]_D^{32}$ = +29.27 ° (c = 1.16, $CH_2Cl_2$)

NMR ($CDCl_3$) δ: 0.89 (6H, m), 1.2-1.5 (18H, m), 1.58 (2H, m), 1.78 (2H, m), 2.47 (1H, dt), 2.65 (1H, ddd), 3.49 (2H, t), 3.59 (1H, dd), 3.68 (1H, dd), 3.97 (2H, t), 4.06 (1H, t), 4.72 (1H, m), 6.94 (2H, d), 7.30 (2H, d), 7.48 (2H, d), 7.52 (2H, d)

Example 3

To a solution of diisopropylamine (505 mg) in tetrahydrofuran (10 ml) cooled at -78 °C is added dropwise a 15 % solution of n-butyllithium in hexane (3 ml) and the temperature is raised gradually to 0 °C and the mixture is stirred for 30 minutes. To this reaction solution is added dropwise a solution of 4-(4′-n-heptyl)-biphenylacetic acid (700 mg) prepared in Preparation 5 in tetrahydrofuran (6 ml) and the mixture is stirred for 1 hour. The reaction solution is cooled to -78 °C and a solution of commercially available (R)-1,2-epoxyheptane ($[\alpha]_D^{25}$ +15.0 ° (neat))(260 mg) in tetrahydrofuran (1 ml) is added dropwise. After the temperature of the reaction solution is gradually raised to room temperature and the reaction solution is stirred for 6 hours, water is added and the mixture is acidified with hydrochloric acid, followed by extraction

of the product with chloroform. To the extract is added dry benzene and a catalytic amount of sulfuric acid and the mixture is stirred with heating for 6 hours while draining benzene in portions. After cooling, the benzene is distilled off under reduced pressure and the residue is purified by silica gel column chromatography to give γ-lactone derivatives (2R, 4R)(330 mg) and (2S, 4R)(383 mg) of the following formulas, respectively.

(2R, 4R) isomer:

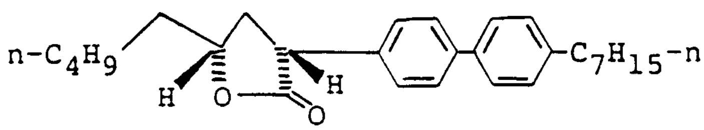

Phase transfer temperature:

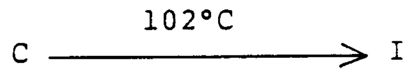


$[\alpha]_D^{27}$ = -5.66° (c = 1.089, $CH_2Cl_2$)

NMR ($CDCl_3$) δ: 0.90 (6H, m), 1.20-1.85 (18H, m), 2.08 (1H, dt), 2.63 (2H, t), 2.78 (1H, m), 3.92 (1H, dd), 4.51 (1H, m), 7.25 (2H, d), 7.34 (2H, d), 7.49 (2H, d), 7.57 (2H, d) (2S, 4R) isomer:

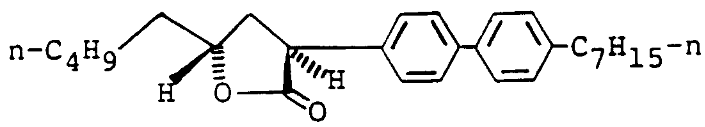

Phase transfer temperature:

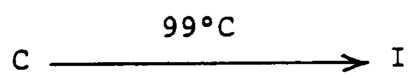


$[\alpha]_D^{28}$ = +33.48° (c = 1.027, $CH_2Cl_2$)

NMR ($CDCl_3$) δ: 0.90 (6H, m), 1.2-1.9 (18H, m), 2.35-2.68 (4H, m), 3.94 (1H, dd), 4.67 (1H, m), 7.25 (2H, d), 7.34 (2H, d), 7.48 (2H, d), 7.57 (2H, d)

Example 4

To a solution of diisopropylamine (505 mg) in tetrahydrofuran (10 ml) cooled at -78°C is added dropwise a 15 % solution of n-butyllithium in hexane (3 ml) and the temperature is raised gradually to 0°C and the mixture is stirred for 30 minutes. To this reaction solution is added dropwise a solution of 4-(4′-n-butyltranscyclohcexyl)phenylaceticacid (600 mg) prepared in Preparation 7 in tetrahydrofuran (3 ml) and the mixture is stirred for 1 hour. The reaction solution is cooled to -78°C and a solution of (S)-allylglycidyl ether (275 mg) prepared in Preparation 4 in tetrahydrofuran (1 ml) is added dropwise. After the temperature of the reaction solution is gradually raised to room temperature and the reaction solution is stirred for 6 hours, water is added and the mixture is acidified with hydrochloric acid, followed by extraction of the product with chloroform. To the extract is added dry benzene and a catalytic amount of sulfuric acid and the mixture is stirred with heating for 6 hours while draining benzene in portions. After cooling, the benzene is distilled off under reduced pressure and the residue is purified by silica gel column chromatography to give γ-lactone derivatives (2S, 4R)(320 mg) and (2R, 4R)(246 mg) of the following formulas, respectively.

(2S, 4R) isomer:

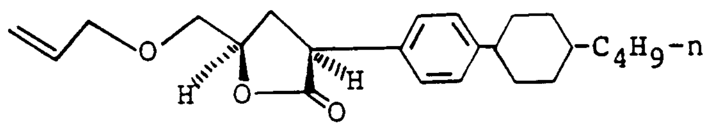

Phase transfer temperature:

$$C \xrightarrow{107^\circ C} I$$

$[\alpha]_D^{28}$ = -3.22° (c = 1.033, $CH_2Cl_2$)
NMR ($CDCl_3$) δ: 0.8-1.5 (14H, m), 1.86 (4H, m), 2.30 (1H, dt), 2.45 (1H, m), 2.68 (1H, m), 3.62-3.76 (2H, m), 3.86 (1H, dd), 4.09 (2H, m), 4.65 (1H, m), 5.22 (1H, m), 5.30 (1H, m), 5.91 (1H, m), 7.20 (4H, s)
(2R, 4R) isomer:

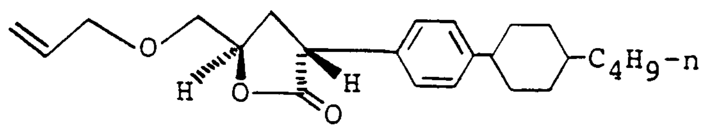

Phase transfer temperature:

$$C \xrightarrow{76^\circ C} I$$

$[\alpha]_D^{28}$ = -40.42° (c = 1.024, $CH_2Cl_2$)
NMR ($CDCl_3$) δ: 0.8-1.5 (14H, m), 1.86 (4H, m), 2.37-2.55 (2H, m), 2.65 (1H, m), 3.60-3.76 (2H, m), 3.95-4.1 (3H, m), 4.75 (1H, m), 5.22 (1H, m), 5.29 (1H, m), 5.91 (1H, m), 7.19 (4H, s)

Example 5

To a solution of diisopropylamine (505 mg) in tetrahydrofuran (10 ml) cooled at -78°C is added dropwise a 15 % solution of n-butyllithium in hexane (3 ml) and the temperature is raised gradually to 0°C and the mixture is stirred for 30 minutes. To this reaction solution is added dropwise a solution of 4-(4'-n-butyltranscyclohexyl)-phenylaceticacid (600 mg) prepared in Preparation 7 in tetrahydrofuran (3 ml) and the mixture is stirred for 1 hour. The reaction solution is cooled to -78°C and a solution of commercially available (R)-1,2-epoxytridecane ($[\alpha]_D^{25}$ +9.8° (neat))(477 mg) in tetrahydrofuran (1 ml) is added dropwise. After the temperature of the reaction solution is gradually raised to room temperature and the reaction solution is stirred for 6 hours, water is added and the mixture is acidified with hydrochloric acid, followed by extraction of the product with chloroform. To the extract is added dry benzene and a catalytic amount of sulfuric acid and the mixture is stirred with heating for 6 hours while draining benzene in portions. After cooling, the benzene is distilled off under reduced pressure and the residue is purified by silica gel column chromatography to give γ-lactone derivatives (2R, 4R) (320 mg) and (2S, 4R)(367 mg) of the following formulas, respectively.
(2R, 4R) isomer:

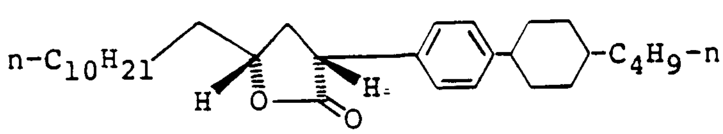

Phase transfer temperature:

$$C \xrightarrow{116^\circ C} I$$

$[\alpha]_D^{26}$ = -3.57° (c = 1.035, $CH_2Cl_2$)
NMR ($CDCl_3$) δ: 0.8-1.9 (41H, m), 2.02 (1H, dt), 2.45 (1H, m), 2.72 (1H, m), 3.83 (1H, dd), 4.47 (1H, m), 7.20 (4H, s) (2S, 4R) isomer:

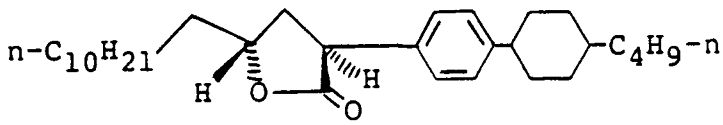

Phase transfer temperature:

$$C \underset{50^\circ C}{\overset{71^\circ C}{\rightleftarrows}} Xn \underset{109^\circ C}{\overset{113^\circ C}{\rightleftarrows}} I$$

$[\alpha]_D^{24}$ = +31.02° (c = 1.038, $CH_2Cl_2$)
NMR ($CDCl_3$) δ: 0.8-1.9 (41H, m), 2.28-2.55 (3H, m), 3.87 (1H, dd), 4.62 (1H, m), 7.19 (4H, s)

<u>Example 6</u>

To a solution of diisopropylamine (505 mg) in tetrahydrofuran (10 ml) cooled at -78°C is added dropwise a 15 % solution of n-butyllithium in hexane (3 ml) and the temperature is raised gradually to 0°C and the mixture is stirred for 30 minutes. To this reaction solution is added dropwise a solution of 4-(4'-n-heptyl)-biphenylacetic acid (700 mg) prepared in Preparation 5 in tetrahydrofuran (3 ml) and the mixture is stirred for 1 hour. The reaction solution is cooled to -78°C and a solution of (R)-methylglycidyl ether (212 mg) prepared in Preparation 2 in tetrahydrofuran (1 ml) is added dropwise. After the temperature of the reaction solution is gradually raised to room temperature and the reaction solution is stirred for 6 hours, water is added and the mixture is acidified with hydrochloric acid, followed by extraction of the product with chloroform. To the extract is added dry benzene and a catalytic amount of sulfuric acid and the mixture is stirred with heating for 6 hours while draining benzene in portions. After cooling, the benzene is distilled off under reduced pressure and the residue is purified by silica gel column chromatography to give γ-lactone derivatives (2R, 4S) (113 mg) and (2S, 4S) (255 mg) of the following formulas, respectively.
(2R, 4S) isomer:

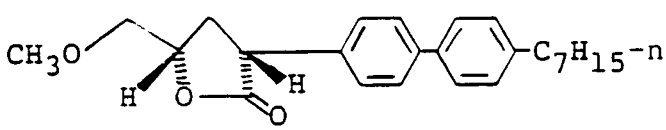

Phase transfer temperature:

$$C \xrightarrow{68^\circ C} I$$

$[\alpha]_D^{21}$ = +0.35° (c = 1.01, $CH_2Cl_2$)
NMR ($CDCl_3$) δ: 0.88 (3H, m), 1.2-1.45 (8H, m), 1.65 (2H, m), 2.34 (1H, dt), 2.6-2.8 (3H, m), 3.46 (3H, s),

3.67 (2H, m), 3.94 (1H, dd), 4.67 (1H, m), 7.25 (2H, d), 7.36 (2H, d), 7.49 (2H, d), 7.58 (2H, d)
(2S, 4S) isomer:

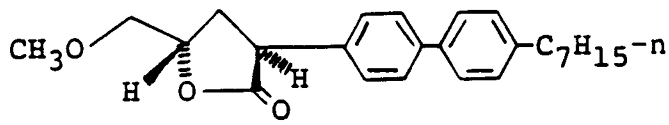

Phase transfer temperature:

$$C \xrightarrow{66°C} I$$

$[\alpha]_D^{23}$ = +34.16° (c = 1.013, $CH_2Cl_2$)
NMR ($CDCl_3$) δ: 0.88 (3H, m), 1.2-1.45 (8H, m), 1.65 (2H, m), 2.52 (1H, dt), 2.59-2.75 (3H, m), 3.44 (3H, s), 3.65 (2H, m), 4.08 (1H, t), 4.77 (1H, m), 7.24 (2H, d), 7.33 (2H, d), 7.48 (2H, d), 7.57 (2H, d)

Example 7

To a solution of diisopropylamine (506 mg) in tetrahydrofuran (10 ml) cooled at -78°C is added dropwise a 15 % solution of n-butyllithium in hexane (3 ml) and the temperature is raised gradually to 0°C and the mixture is stirred for 30 minutes. To this reaction solution is added dropwise a solution of 2-(4'-nonyloxy-4-biphenyl)propionic acid (730 mg) prepared in Preparation 8 in tetrahydrofuran (8 ml) and the mixture is stirred for 1 hour. The reaction solution is cooled to -78°C and a solution of (R)-1,2-epoxynonane (312 mg) prepared in Preparation 1 in tetrahydrofuran (1 ml) is added dropwise. After the temperature of the reaction solution is gradually raised to room temperature and the reaction solution is stirred for 6 hours, water is added and the mixture is acidified with hydrochloric acid, followed by extraction of the product with chloroform. To the extract is added dry benzene and a catalytic amount of sulfuric acid and the mixture is stirred with heating for 6 hours while draining benzene in portions. After cooling, the benzene is distilled off under reduced pressure and the residue is purified by silica gel column chromatography to give γ-lactone derivatives (2R, 4R) (408 mg) and (2S, 4R) (280 mg) of the following formulas, respectively.
(2R, 4R) isomer:

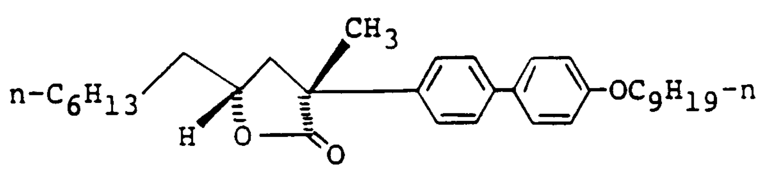

Phase transfer temperature:

$$C \xrightarrow{95°C} I$$

$[\alpha]_D^{25}$ = +13.24° (c = 1.06, $CH_2Cl_2$)
NMR ($CDCl_3$) δ: 0.88 (6H, m), 1.2-1.7 (24H, m), 1.67 (3H, s), 1.79 (2H, m), 2.33 (1H, dd), 2.50 (1H, dd), 3.99 (2H, t), 4.57 (1H, m), 6.96 (2H, d), 7.45-7.55 (6H, m)
(2S, 4R) isomer:

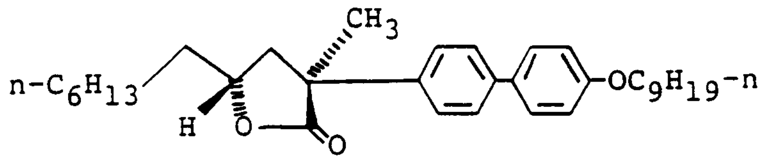

Phase transfer temperature:

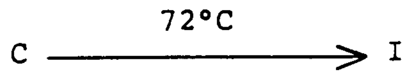

$[\alpha]_D^{23}$ = +25.11° (c = 1.017, $CH_2Cl_2$)
NMR ($CDCl_3$) δ: 0.88 (6H, m), 1.15-1.70 (24H, m), 1.61 (3H, s), 1.78 (2H, m), 1.99 (1H, dd), 2.77 (1H, dd), 3.99 (2H, t), 4.24 (1H, m), 6.96 (2H, d), 7.35-7.60 (6H, m)

Example 8

To a solution of diisopropylamine (505 mg) in tetrahydrofuran (10 ml) cooled at -78°C is added dropwise a 15 % solution of n-butyllithium in hexane (3 ml) and the temperature is raised gradually to 0°C and the mixture is stirred for 30 minutes. To this reaction solution is added dropwise a solution of commercially available 4-octyloxy0henylacetic acid (594 mg) in tetrahydrofuran (3 ml) and the mixture is stirred for 1 hour. The reaction solution is cooled to -78°C and a solution of (R)-1,2-epoxynonane (320 mg) prepared in Preparation 1 in tetrahydrofuran (1 ml) is added dropwise. After the temperature of the reaction solution is gradually raised to room temperature and the reaction solution is stirred for 6 hours, water is added and the mixture is acidified with hydrochloric acid, followed by extraction of the product with chloroform. To the extract is added dry benzene and a catalytic amount of sulfuric acid and the mixture is stirred with heating for 6 hours while draining benzene in portions. After cooling, the benzene is distilled off under reduced pressure and the residue is purified by silica gel column chromatography to give γ-lactone derivatives (2R, 4R) (289 mg) and (2S, 4R) (285 mg) of the following formulas, respectively.
(2R, 4R) isomer:

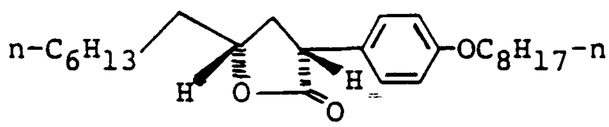

Phase transfer temperature:

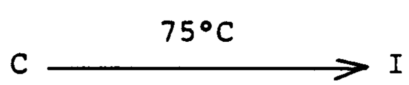

$[\alpha]_D^{26}$ = -3.72° (c = 1.018, $CH_2Cl_2$)
NMR ($CDCl_3$) δ: 0.89 (6H, m), 1.2-1.9 (24H, m), 1.99 (1H, dt), 2.71 (1H, ddd), 3.81 (1H, dd), 3.94 (2H, t), 4.46 (1H, m), 6.88 (2H, d), 7.81 (2H, d)
(2S, 4R) isomer:

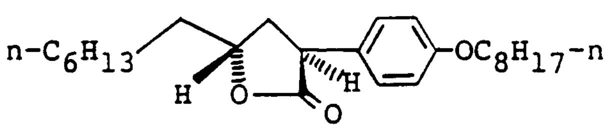

Phase transfer temperature:

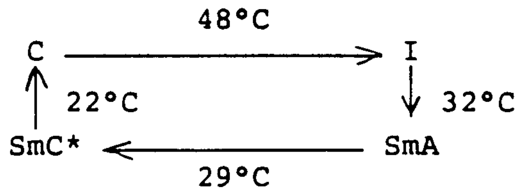


$[\alpha]_D^{25}$ = +34.23° (c = 1.00, $CH_2Cl_2$)
NMR ($CDCl_3$) δ: 0.89 (6H, m), 1.2-1.9 (24H, m), 2.3-2.55 (2H, m), 3.85 (1H, dd), 3.94 (2H, t), 4.61 (1H, m), 6.88 (2H, d), 7.18 (2H, d)

Example 9

To a solution of diisopropylamine (505 mg) in tetrahydrofuran (10 ml) cooled at -78°C is added dropwise a 15 % solution of n-butyllithium in hexane (3 ml) and the temperature is raised gradually to 0°C and the mixture is stirred for 30 minutes. To this reaction solution is added dropwise a solution of 4-(4'-n-heptyl)-biphenylacetic acid (682 mg) prepared in Preparation 5 in tetrahydrofuran (3 ml) and the mixture is stirred for 1 hour. The reaction solution is cooled to -78°C and a solution of (S)-n-hexylglycidyl ether (445 mg) prepared in Preparation 3 in tetrahydrofuran (1 ml) is added dropwise. After the temperature of the reaction solution is gradually raised to room temperature and the reaction solution is stirred for 6 hours, water is added and the mixture is acidified with hydrochloric acid, followed by extraction of the product with chloroform. To the extract is added dry benzene and a catalytic amount of sulfuric acid and the mixture is stirred with heating for 6 hours while draining benzene in portions. After cooling, the benzene is distilled off under reduced pressure and the residue is purified by silica gel column chromatography to give γ-lactone derivatives (2S, 4R) (401 mg) and (2R, 4R) (465 mg) of the following formulas, respectively.
(2S, 4R) isomer:

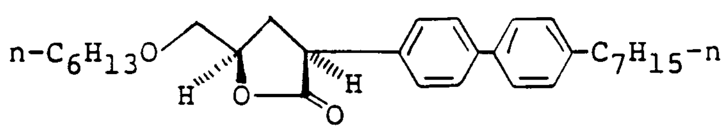

Phase transfer temperature:

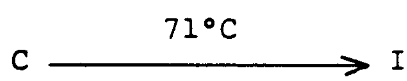


$[\alpha]_D^{22}$ = -2.17° (c = 1.07, $CH_2Cl_2$)
NMR ($CDCl_3$) δ: 0.86-0.91 (6H, m), 1.29-1.61 (18H, m), 2.28-2.42 (1H, m), 2.61-2.76 (3H, m), 3.52 (2H, t, J = 6.60 Hz), 3.61-3.75 (2H, m), 3.92 (1H, dd, J = 9.16 Hz, 12.09 Hz), 4.62-4.67 (1H, m), 7.24 (2H, d, J = 8.06 Hz), 7.35 (2H, d, J = 8.42 Hz), 7.48 (2H, d, J = 8.42 Hz), 7.57 (2H, d, J = 8.06 Hz)
(2R, 4R) isomer:

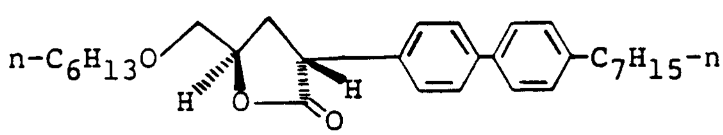

Phase transfer temperature:

$$C \xrightarrow{48^\circ C} I$$

$[\alpha]_D^{22}$ = -37.95° (c = 1.003, $CH_2Cl_2$)

NMR ($CDCl_3$) δ: 0.86-0.90 (6H, m), 1.29-1.60 (18H, m), 2.45-2.57 (1H, m), 2.61-2.74 (3H, m), 3.51 (2H, t, J = 6.68 Hz), 3.60-3.75 (2H, m), 4.09 (1H, t, J = 9.35 Hz), 4.74-4.78 (1H, m), 7.24 (2H, d, J = 8.06 Hz), 7.33 (2H, d, J = 8.43 Hz), 7.48 (2H, d, J = 8.43 Hz), 7.57 (2H, d, J = 8.06 Hz)

<Liquid crystal compositions containing the compound (I) and physical properties thereof>

Example 10

The (2R, 4R) isomer of the γ-lactone derivative prepared in Example 1 of the following formula:

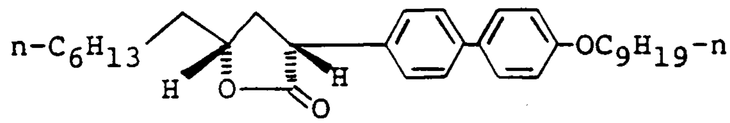

and a compound of the following formula:

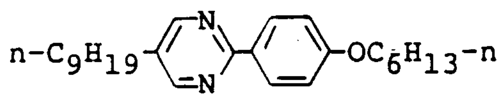

are mixed together at a weight ratio of 1 : 19 to give a liquid crystal composition.

The obtained liquid crystal composition is measured for the response speed. As a result, it is found that the composition shows the response speed as high as 85 μsec (30°C). The response speed is measured in such a way that the above composition is sealed in a cell (thickness of spacer: 2 μm) surface-treated with an aligning agent and a change in strength of transmitted light is measured when the cell is charged with a voltage of $V_{p-p}$ = 20 V with use of crossed nicols, wherein PET (polyethylene terephthalate) film as the spacer, polyimide film as the aligning agent and ITO (indium-tin oxide) electrode are employed and the rubbing is made in parallel direction.

Example 11

Using the (2S, 4S) isomer of the γ-lactone derivative prepared in Example 2 of the following formula:

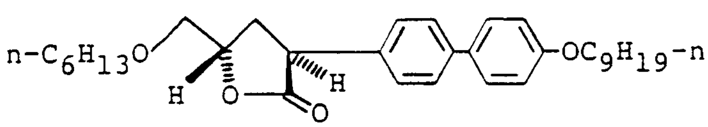

liquid crystal composition is prepared comprising the following components.

The obtained composition is measured on the response speed in the same manner as described in Example 10. As a result, it is found that the above (2S, 4S) isomer of γ-lactone derivative alone does not show the ferroelectricity but is shows the ferroelectricity when it is mixed with other liquid crystalline compound.

| Liquid crystalline compound | wt % |
| --- | --- |
| γ-Lactone deriv. (2S, 4S) isomer (Ex. 2) | 5.0 |
| 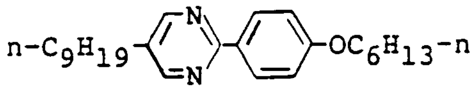 | 23.7 |
| 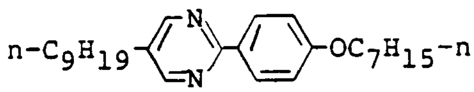 | 23.7 |
| 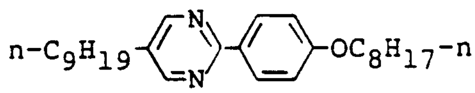 | 23.8 |
| 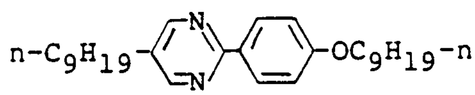 | 23.8 |

Phase transfer temperature:

$$C \underset{-2°C}{\overset{18°C}{\rightleftarrows}} SmC^* \underset{48°C}{\overset{53°C}{\rightleftarrows}} SmA \underset{65°C}{\overset{68°C}{\rightleftarrows}} I$$

Response speed: 320 μsec (40 °C)

## Claims

**1.** A liquid crystalline compound with an optically active γ-lactone ring having the following formula (I):

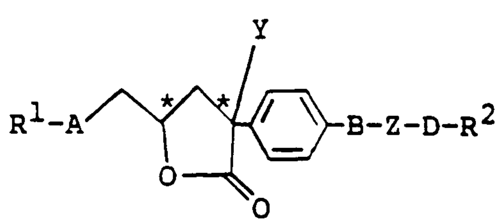

(I)

wherein $R^1$ and $R^2$ are each independently an alkyl group having 1 to 15 carbon atoms or an alkenyl group having 2 to 15 carbon atoms; A and D are each independently a single bond or -O-; B is a single bond or

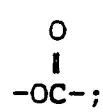

Z is a single bond or one of the groups

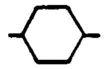 or 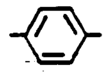;

Y is hydrogen atom or $-CH_3$; and the symbol * is an asymmetric carbon atom with the proviso that the compound in which A is O, $R^1$ is $-CH_3$, Y is a hydrogen atom, B and Z are each a single bond, D is O and $R^2$ is $-CH_3$ is excluded.

2. The compound according to claim 1 which is in the form of a racemic mixture.

3. A liquid crystal composition which comprises at least one of the liquid crystalline compounds (I) as set forth in claim 1 or 2, and other ferroelectric liquid crystals or non-chiral liquid crystals or a mixture thereof.

4. The composition according to claim 3, wherein the chiral or non-chiral liquid crystal to be mixed with the compound of the formula (I) is a compound of the general formula (J):

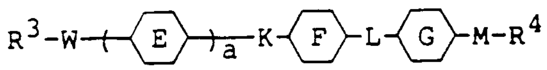 (J)

wherein E, F and G are each independently one of the 6-membered rings

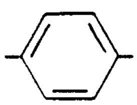, 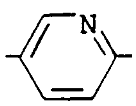, 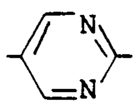, 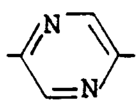, 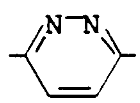, 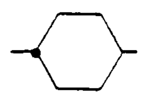,

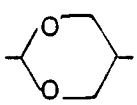 and 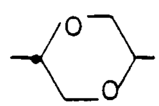,

the hydrogen atom(s) in the 6-membered ring being optionally substituted with a halogen atom, cyano group or nitro group; a is 0 or 1; W, K, L and M are each a single bond or one of the groups

$$-\overset{O}{\overset{\|}{C}}-O-,$$

$-CH_2O-$, $-C{\equiv}C-$, $-CH=CH-$, -O-, -S-,

$$-CH=CH-\overset{O}{\overset{\|}{C}}-O-, \quad -O-\overset{O}{\overset{\|}{C}}-O-,$$

$-N=CH-$ and $-CH_2-CH_2-$; provided that K is a single bond when $a=0$; $R^3$ and $R^4$ are each independently a straight chain or branched chain alkyl group having 1 to 15 carbon atoms, which may contain one or more asymmetric carbon atoms.

5. The composition according to claim 3, wherein the chiral or non-chiral liquid crystal to be mixed with the compound of the formula (I) is at least one compound of the formula (J-1):

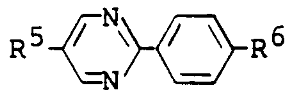

(J-1)

wherein $R^5$ and $R^6$ are the same or different and are each a straight chain or branched chain alkyl group having 1 to 15 carbon atoms or a straight chain or branched chain alkoxy group having 1 to 15 carbon atoms, said alkyl and alkoxy groups having optionally one or more asymmetric carbon atoms, or of the formula (J-2):

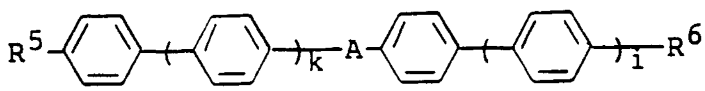

(J-2)

wherein $R^5$ and $R^6$ are as defined above, A is

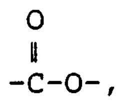

and k and i are independently 0 or 1, but $k + i \neq 2$.

**6.** An element for opto-electronics devices comprising the liquid crystal composition as set forth in any one of claims 3 to 5.

**Patentansprüche**

**1.** Flüssigkristalline Verbindung mit einem optisch aktivem $\gamma$-Lactonring der Formel (I):

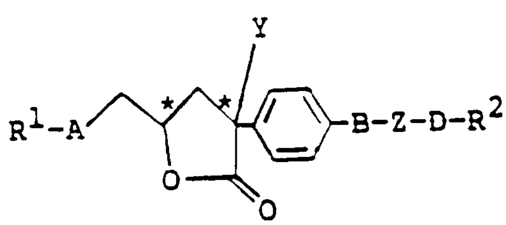

(I)

in der $R^1$ und $R^2$ jeweils unabhängig voneinander einen Alkylrest mit 1 bis 15 Kohlenstoffatomen oder einen Alkenylrest mit 2 bis 15 Kohlenstoffatomen bedeuten; A und D jeweils unabhängig voneinander eine Einfachbindung oder die Gruppe -O- darstellen; B eine Einfachbindung oder

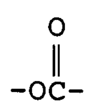

bedeutet; Z eine Einfachbindung oder eine der Gruppen

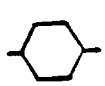

oder

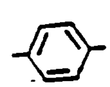

darstellt;

Y ein Wasserstoffatom oder die Gruppe $-CH_3$ darstellt; und das Symbol * ein asymmetrisches Kohlenstoffatom bedeutet, mit der Maßgabe, daß die Verbindung, in der A ein Sauerstoffatom bedeutet, $R^1$ die Gruppe $-CH_3$ darstellt, Y ein Wasserstoffatom bedeutet, B und Z jeweils eine Einfachbindung darstellen, D ein Sauerstoffatom bedeutet, und $R^2$ die Gruppe $-CH_3$ darstellt, ausgenommen ist.

**2.** Verbindung nach Anspruch 1, vorliegend als razemisches Gemisch.

**3.** Flüssigkristallzusammensetzung, umfassend mindestens eine der flüssigkristallinen Verbindungen (I) nach Anspruch 1 oder 2 und andere ferroelektrische Flüssigkristalle oder nicht-chirale Flüssigkristalle oder ein Gemisch davon.

**4.** Zusammensetzung nach Anspruch 3, wobei der chirale oder nicht-chirale Flüssigkristall der mit der Verbindung der Formel (I) vermischt wird, eine Verbindung der allgemeinen Formel (J) ist:

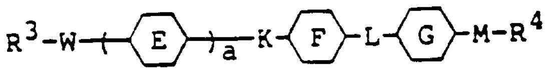

(J)

in der E, F und G unabhängig voneinander einen der 6-gliedrigen Ringe

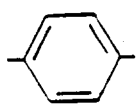, 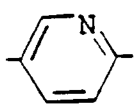, 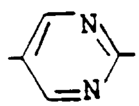, 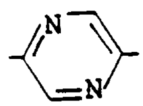, 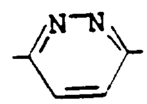, 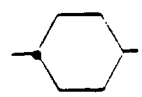,

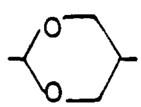 und 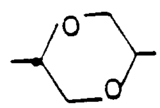,

bedeuten, wobei das (die) Wasserstoffatom(e) in dem 6-gliedrigen Ring gegebenenfalls durch ein Halogenatom, eine Cyano- oder Nitrogruppe substituiert ist (sind); a 0 oder 1 bedeutet; W, K, L und M jeweils eine Einfachbindung oder eine der Gruppen

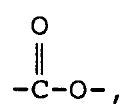

$-CH_2O-$, $-C{\equiv}C-$, $-CH=CH-$, $-O-$, $-S-$,

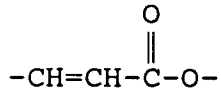

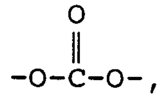

-N=CH- und $-CH_2-CH_2-$ bedeuten; mit der Maßgabe, daß K eine Einfachbindung darstellt, wenn a gleich 0 ist; und $R^3$ und $R^4$ jeweils unabhängig voneinander einen geradkettigen oder verzweigtkettigen Alkylrest mit 1 bis 15 Kohlenstoffatomen bedeutet, der eines oder mehrere asymmetrische Kohlenstoffatome enthalten kann.

**5.** Zusammensetzung nach Anspruch 3, wobei der chirale oder nicht-chirale Flüssigkristall, der mit der Verbindung der Formel (I) vermischt wird, mindestens eine Verbindung der Formel (J-1):

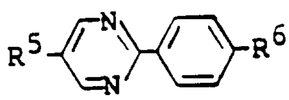

(J-1)

in der $R^5$ und $R^6$ gleich oder verschieden sind und jeweils einen gerad- oder verzweigtkettigen Alkylrest mit 1 bis 15 Kohlenstoffatomen oder einen geradkettigen oder verzweigtkettigen Alkoxyrest mit 1 bis 15 Kohlenstoffatomen bedeuten, wobei die Alkyl- und Alkoxyreste gegebenenfalls eines oder mehrere asymmetrische Kohlenstoffatome aufweisen,
oder der Formel (J-2) ist:

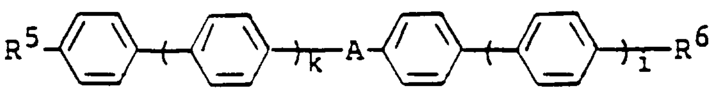

(J-2)

in der $R^5$ und $R^6$ wie vorstehend definiert sind, A die Gruppe

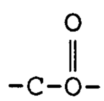

darstellt und k und i unabhängig voneinander 0 oder 1 darstellen, wobei jedoch k + i ≠ 2 ist.

**6.** Element für optoelektronische Bauelemente, umfassend eine Flüssigkristallzusammensetzung nach einem der AnSprüche 3 bis 5.

## Revendications

**1.** Composé cristallino-liquide ayant un cycle γ-lactone optiquement actif, répondant à la formule (I) suivante :

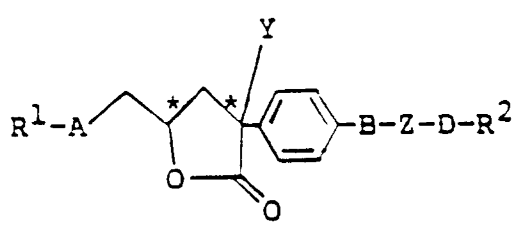

(I)

dans laquelle $R^1$ et $R^2$ sont chacun indépendamment un groupe alkyle ayant 1 à 15 atomes de carbone ou un groupe alcényle ayant 2 à 15 atomes de carbone ; A et D sont chacun indépendamment une simple liaison ou -O- ; B est une simple liaison ou

O
||
-OC- ;

Z est une simple liaison ou un des

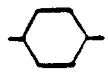 ou 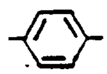 ;

groupes Y est un atome d'hydrogène ou $-CH_3$ ; et le symbole * désigne un atome de carbone asymétrique, sous réserve que le composé dans lequel A est O, $R^1$ est $-CH_3$, Y est un atome d'hydrogène, B et Z sont chacun une simple liaison, D est O et $R^2$ est $-CH_3$ soit exclu.

**2.** Composé selon la revendication 1 qui est sous forme d'un mélange racémique.

**3.** Composition de cristaux liquides qui comprend au moins un des composés liquido-cristallins (I), comme indiqué dans la revendication 1 ou 2, et d'autres cristaux liquides ferroélectriques ou des cristaux liquides non chiraux ou un de leurs mélanges.

**4.** Composition selon la revendication 3, dans laquelle le cristal liquide chiral ou non chiral à mélanger avec le composé de formule (I) est un composé de formule générale (J) :

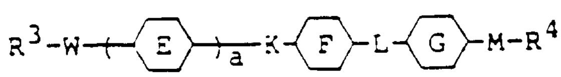 (J)

dans laquelle E, F et G sont chacun indépendamment un des cycles à 6 chaînons

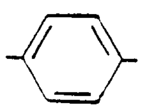, 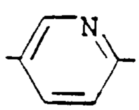, 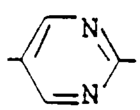, 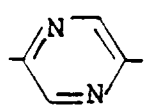, 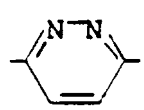, 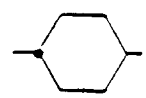,

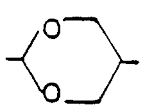 et 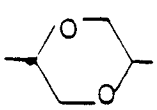,

le ou les atomes d'hydrogène du cycle à 6 chaînons étant facultativement substitués avec un atome d'halogène, un groupe cyano ou un groupe nitro ; a est 0 ou 1 ; W, K, L et M sont chacun une simple liaison ou un des groupes

O
||
-C-O-,

$-CH_2O-$, $-C\equiv C-$, $-CH=CH-$, -O-, -S-,

O O
|| ||
-CH=CH-C-O-, -O-C-O-,

-N=CH- et $-CH_2-CH_2-$ ; sous réserve que K soit une simple liaison lorsque A = 0 ; et $R^3$ et $R^4$ sont chacun indépendamment un groupe alkyle à chaîne droite ou ramifiée ayant 1 à 15 atomes de

carbone, pouvant contenir un ou plusieurs atomes de carbone asymétriques.

**5.** Composition selon la revendication 3, dans laquelle le cristal liquide chiral ou non chiral à mélanger avec le composé de formule (I) est au moins un composé de formule (J-1) :

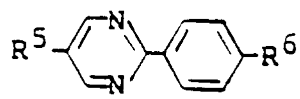

(J-1)

dans laquelle $R^5$ et $R^6$ sont identiques ou différents et sont chacun un groupe alkyle à chaîne droite ou ramifiée ayant 1 à 15 atomes de carbone ou un groupe alcoxy à chaîne droite ou ramifiée ayant 1 à 15 atomes de carbone, lesdits groupes alkyle et alcoxy ayant facultativement un ou plusieurs atomes de carbone asymétriques, ou de formule (J-2) :

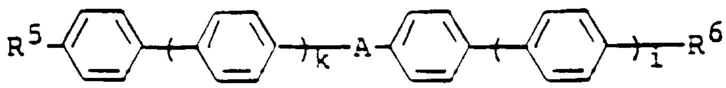

(J-2)

dans laquelle $R^5$ et $R^6$ sont définis comme ci-dessus, A est

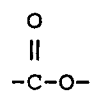

et k et i sont indépendamment 0 ou 1, mais $k + i \neq 2$.

**6.** Elément pour dispositifs optoélectroniques comprenant la composition de cristaux liquides selon l'une quelconque des revendications 3 à 5.